# EUROPÄISCHE PATENTSCHRIFT

(11) **EP 3 083 911 B1**
(45) Veröffentlichungstag und Bekanntmachung des Hinweises auf die Patenterteilung: **21.03.2018**
(21) Anmeldenummer: 14812176.7
(22) Anmeldetag: 08.12.2014
(51) Int. Cl.: C11B 9/00, A61Q 13/00, C09J 11/06, C11D 3/50

(54) **MICHAEL-SYSTEME FÜR DIE DUFTSTOFF-STABILISIERUNG**
MICHAEL SYSTEMS FOR ODOR STABILIZATION
SYSTÈMES DE MICHAEL POUR LA STABILISATION D'UNE MATIÈRE ODORIFÉRANTE

(30) Priorität: 17.12.2013 DE 102013226216
(43) Veröffentlichungstag der Anmeldung: 26.10.2016
(73) Patentinhaber: Henkel AG & Co. KGaA, 40589 Düsseldorf (DE)
(72) Erfinder: HUCHEL, Ursula, 50733 Köln (DE); BARON, Lukas, 47447 Moers-Kapellen (DE); URLICHS, Stefan, 45309 Essen (DE); MATERNE, Manuela, 41564 Kaarst (DE)
(74) Vertreter: Viering, Jentschura & Partner mbB Patent- und Rechtsanwälte
(86) Internationale Anmeldenummer: PCT/EP2014/076878
(87) Internationale Veröffentlichungsnummer: WO 2015/091054

(56) Entgegenhaltungen:
- EP-A1- 1 552 814
- EP-A1- 2 832 440
- EP-A2- 2 524 704
- CH-A- 529 829
- US-A- 3 769 328
- US-A- 3 819 712
- US-A1- 2007 042 934
- Barahman Movassagh ET AL: "Michael addition of thiols to alpha, beta-unsaturated carbonyl compounds under solvent-free conditions", ARKIVOC, Bd. 2006, Nr. xii 31. Dezember 2006 (2006-12-31), Seiten 130-137, XP055174513, ISSN: 1424-6376 Gefunden im Internet: URL:http://www.arkat-usa.org/get-file.php? fileid=22732 [gefunden am 2015-03-06]

## Beschreibung

Die vorliegende Erfindung betrifft eine Riechstoffzusammensetzung enthaltend (i) mindestens eine α,β-ungesättigte Verbindung ausgewählt aus α,β-ungesättigten Ketonen oder aus α,β-ungesättigten Aldehyden oder aus Mischungen daraus und (ii) mindestens einen Thiolgruppen-haltigen Riechstoff, ein Mittel, das diese Riechstoffzusammensetzung enthält, sowie die Verwendung eines Wasch-, Reinigungs- oder Pflegemittels enthaltend eine ebensolche Riechstoffzusammensetzung.

Wasch- oder Reinigungsmittel, kosmetische Mittel, wie Deodorants, bzw. Luftpflegemittel (z.B. Raumlufterfrischer, Raumdeodorant, Raumspray usw.), aber auch Klebstoffe enthalten zumeist Duftstoffe, die den Mitteln einen angenehmen Geruch verleihen und gleichzeitig Schlechtgerüche olfaktorisch überdecken sollen. Die Duftstoffe maskieren dabei zumeist auch den Geruch anderer Inhaltstoffe, so dass beim Verbraucher ein angenehmer Geruchseindruck entsteht.

Wirkstoffe, wie beispielsweise Riechstoffe, können auf Grund Ihrer Struktur und ihren enthaltenen, funktionellen Gruppen Reaktionen mit in der Umgebung befindlichen Stoffen eingehen, die einen unerwünschten Abbau dieser Wirkstoffe bewirken. Dieser Abbau kann in Form von Wechselwirkungen mit einer Produktmatrix stattfinden, bei der eigentlichen Anwendung des Produktes sowie durch Reaktionen, hervorgerufen durch Umweltbedingungen, speziell durch Licht, Wärme, Sauerstoff, Wasser und Protonen, hervorgerufen werden. Besonders instabil sind solche Riechstoffe, die reaktive funktionelle Gruppen tragen, beispielsweise Thiole. Durch den unerwünschten Abbau der Riechstoffe vermindert sich der Geruchseindruck und dessen Intensität.

Aufgabe der vorliegenden Erfindung war es, eine Riechstoffzusammensetzung bereitzustellen, die den Abbau von Thiolgruppen-haltigen Wirkstoffen vermindert.

Gelöst wird diese Aufgabe durch den Gegenstand der Erfindung, nämlich durch eine Riechstoffzusammensetzung, die (i) mindestens ein α,β-ungesättigtes Keton oder Aldehyd und (ii) mindestens einen Thiolgruppen-haltigen Riechstoff enthält sowie ein Mittel, das diese Riechstoffzusammensetzung enthält, wobei die mindestens eine α,β-ungesättigte Verbindung (i) und der mindestens eine Thiolgruppen-haltige Riechstoff (ii) im molaren Verhältnis zwischen 5:1 und 1:1 eingesetzt werden. Es konnte überraschend gefunden werden, dass die erfindungsgemäße Riechstoffzusammensetzung den unerwünschten Abbau der Thiolgruppen-haltigen Wirkstoffe vermindert und somit zu einer Intensitäts- und Wirkungssteigerung des Wirkstoffs führt. Ferner sind α,β-ungesättigte Ketone im Gegensatz zu vielen üblichen Stabilisatoren für Wirkstoffe, wie UV Absorber (beispielsweise Benzotriazole) und Antioxidantien (beispielsweise gehinderte Phenole, Hydroperoxidtypen), oftmals preisgünstig, toxikologisch unbedenklich und für die Anwendung in Wasch- und Reinigungsmittel sowie Kosmetika und Klebstoffe geeignet.

In einem ersten Aspekt richtet sich die vorliegende Erfindung daher auf eine Riechstoffzusammensetzung enthaltend
(i) mindestens eine α,β-ungesättigte Verbindung ausgewählt aus α,β-ungesättigten Ketonen, α,β-ungesättigten Aldehyden oder aus Mischungen daraus,
   und
(ii) mindestens einen Thiolgruppen-haltigen Riechstoff, wobei die mindestens eine α,β-ungesättigte Verbindung (i) und der mindestens eine Thiolgruppen-haltige Riechstoff (ii) im molaren Verhältnis zwischen 5:1 und 1:1 eingesetzt werden.
Die zählweise der α,β-Position der ungesättigten Stelle im Molekül startet an der Carbonylgruppe des Ketons beziehungsweise des Aldehyds.

In einem zweiten Aspekt richtet sich die vorliegende Erfindung auf ein Mittel, wobei das Mittel ein Wasch- oder Reinigungsmittel, Luftpflegemittel, kosmetisches Mittel oder ein Klebstoff ist, enthaltend mindestens eine Riechstoffzusammensetzung wie oben definiert, bevorzugt in einer Gesamtmenge von 0,001-99,000 Gew.-%, besonders bevorzugt von 0,05-1,00 Gew.-%, bezogen auf das Gewicht des Wasch-, Reinigungs-, Luftpflegemittels, kosmetischen Mittels oder des Klebstoffs.

In einem weiteren Aspekt betrifft die vorliegende Erfindung die Verwendung eines Wasch- oder Reinigungsmittels, wobei besagte Mittel eine Zusammensetzung nach der Erfindung enthalten, zum Reinigen und/oder Konditionieren von textilen Flächengebilden, insbesondere in einer automatischen Waschmaschine.

Schließlich betrifft die vorliegende Erfindung auch die Verwendung einer α,β-ungesättigten Verbindung ausgewählt aus α,β-ungesättigten Ketonen, α,β-ungesättigten Aldehyden oder aus Mischungen daraus zur Stabilisierung eines Thiolgruppen-haltigen Riechstoffs, beispielsweise in einem Mittel, dass beide Verbindungen enthält.

Alle im Zusammenhang mit den hierin beschriebenen Mitteln angegeben Mengenangaben beziehen sich, sofern nichts anderes angegeben ist, auf Gew.-% jeweils bezogen auf das Gesamtgewicht des Mittels. Des Weiteren beziehen sich derartige Mengenangaben, die sich auf mindestens einen Bestandteil beziehen, immer auf die Gesamtmenge dieser Art von Bestandteil, die im Mittel enthalten ist, sofern nicht explizit etwas anderes angegeben ist. Das heißt, dass sich derartige Mengenangaben, beispielsweise im Zusammenhang mit "mindestens ein Riechstoff", auf die Gesamtmenge von Riechstoffen die im Mittel enthalten ist, beziehen.

"Mindestens ein", wie hierin verwendet, bezieht sich auf 1 oder mehr, beispielsweise 1, 2, 3, 4, 5, 6, 7, 8, 9 oder mehr. Im Zusammenhang mit Bestandteilen der hierin beschriebenen Zusammensetzungen bezieht sich diese Angabe nicht auf die absolute Menge an Molekülen sondern auf die Art des Bestandteils. "Mindestens ein Riechstoff" bedeutet daher beispielsweise ein oder mehrere verschiedene Riechstoffe, d.h. eine oder mehrere verschiedene Arten von Riechstoffen. Zusammen mit Mengenangaben beziehen sich die Mengenangaben auf die Gesamtmenge der entsprechend bezeichneten Art von Bestandteil, wie bereits oben definiert.

Die Begriffe "Riechstoff" und "Duftstoff" werden hierin austauschbar verwendet und bezeichnen insbesondere solche Stoffe, die einen von Menschen als angenehm empfundenen Duft haben.

In einer bevorzugten Ausführungsform der vorliegenden Erfindung ist die mindestens eine α,β-ungesättigte Verbindung eine Verbindung der Formel (I),

R¹-CH=CH-C(=O)R² (I)

wobei R¹ und R² jeweils unabhängig voneinander für Wasserstoff, einen substituierten oder unsubstituierten Alkyl-, Cycloalkyl-, Alkenyl-, Cycloalkenyl-, Aryl- oder Acylgruppen-haltigen Rest oder ein Polymer stehen. Hierbei kann der eine substituierte oder unsubstituierte Alkyl-, Cycloalkyl-, Alkenyl-, Cycloalkenyl- Aryl- oder Acylgruppen-haltigen Rest bis zu 26 Kohlenstoffatome umfassen.

In verschiedenen bevorzugten Ausführungsformen der vorliegenden Erfindung ist die mindestens eine Verbindung der Formel (I) ein Riechstoff.

In weiteren bevorzugten Ausführungsformen der vorliegenden Erfindung steht
(1) R¹ für einen substituierten oder unsubstituierten Alkylrest mit 1 bis 10 Kohlenstoffatomen, bevorzugt für einen Alkylrest mit 1 bis 5 Kohlenstoffatomen, insbesondere für einen Methylrest, und/oder
(2) R² für einen Alkyl-, Cycloalkyl-, Alkenyl-, Cycloalkenyl-, Aryl-, Acylgruppen-haltigen Rest mit wenigstens 5 Kohlenstoffatomen, insbesondere einen cyclischen, substituierten Alkenyl-Rest mit wenigstens 5 Kohlenstoffatomen, vorzugsweise

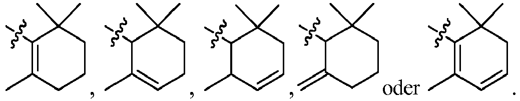

In einer noch weiteren bevorzugten Ausführungsform der vorliegenden Erfindung wird die Verbindung der Formel (I) ausgewählt aus mindestens einem Vertreter der Gruppe die gebildet wird aus α-Damascon, β-Damascon, δ-Damascon, γ-Damascon, Damascenon, Zimtaldehyd, Hydroxyzimtaldehyd, Iron, Citral, Janonen (beispielsweise α-Janon, β-Janon, γ-Janon), α-Isomethylionon, Methylcedrylketon.

Das Polymer, für das R¹ bzw. R² stehen, kann jedes Polymer sein, wie beispielsweise, aber nicht beschränkt darauf, Polyethylen, Polypropylen, Polyvinylchlorid, Polytetrafuoroethylen, Polyester, wie Polycarbonate und Polyethylenetrephthalate, Polyethylenglykol, Polyurethan, Polyethylenglykol und Poly(organo)siloxane. Weitere Polymere können auch Biopolymere und Harze sein. Die erfindungsgemäßen α,β-ungesättigten Ketone und Aldehyde, beziehungsweise die Verbindungen der Formel (I), können miteinander kovalent verknüpft sein, beispielsweise über eine substituierte oder unsubstituierte Alkyl- oder Alkenylkette, so dass diese selbst ein Polymer ausbilden. Solche Ketten können anhand im Stand der Technik bekannter Reaktionen, wie beispielsweise Polymerisationsreaktionen, ausgebildet werden.

Der mindestens eine Thiolgruppen-haltige Riechstoff kann jeder Riechstoff sein, der mindestens eine Thiolgruppe enthält. In einer bevorzugten Ausführungsform der vorliegenden Erfindung ist der Thiolgruppen-haltige Riechstoff ein Riechstoff mit 5-20 Kohlenstoffatomen, bevorzugt (*R*)-, (*S*)-Thioterpineol (p-Menth-1-en-8-thiol), 2-(1-Mercapto-1-methylethyl)-5-methyl-cyclohexanon, 4-Methoxy-2-methyl-2-butanthiol, 3-mercapto-1-hexanol, 2-Mercaptopropansäure, 2-Mercaptopropansäureethylester, 3-(Methylthio)propylmercaptoacetate.

In der vorliegenden Erfindung enthält die Riechstoffzusammensetzung eine Verbindung der Formel (I) und die mindestens eine Thiol-haltige Verbindung im molaren Verhältnis zwischen 5:1 und 1:1, bevorzugt zwischen 3:1 und 1:1.

Der Riechstoffzusammensetzung können weitere Riechstoffe zugesetzt werden, z.B. können synthetische Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan , zu den Aldehyden z.B. die linearen Alkanale mit 8 - 18 C-Atomen, Citronellal, Citronellyloxyacetaldehyd, Cyclamenaldehyd, Lilial und Bourgeonal, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden Mischungen verschiedener Riechstoffe verwendet, die gemeinsam eine ansprechende Duftnote erzeugen.

Solche Mischungen können natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl.

Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Die allgemeine Beschreibung der einsetzbaren Duftstoffe (siehe oben) stellt allgemein die unterschiedlichen Substanzklassen von Riechstoffen dar. Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Ferner können im Rahmen der vorliegenden Erfindung haftfeste Riechstoffe optional eingesetzt warden, beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höher siedenden bzw. festen Riechstoffe natürlichen oder synthetischen Ursprungs können im Rahmen der vorliegenden Erfindung als haftfeste Riechstoffe bzw. Riechstoffgemische eingesetzt werden. Zu diesen Verbindungen zählen die nachfolgend genannten Verbindungen sowie Mischungen aus diesen: Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtalkohol, Indol, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanillin, Veratrumaldehyd, Zimtalkohol, Zimtsäure, Zimtsäureethylester, Zimtsäurebenzylester.

Die Riechstoffzusammensetzungen, die hierin beschrieben werden können in verschiedenen Mitteln enthalten sein, die ebenfalls einen Gegenstand der Erfindung bilden. Derartige Mittel schließen ein, sind aber nicht beschränkt auf Wasch-, Reinigungs-, Luftpflegemittel, kosmetische Mittel sowie Kleber.

Die Waschmittel können solche für Textilien, Teppiche oder Naturfasern sein. Zu den Waschmitteln im Rahmen der Erfindung zählen ferner Waschhilfsmittel, die bei der manuellen oder maschinellen Textilwäsche zum eigentlichen Waschmittel zudosiert werden, um eine weitere Wirkung zu erzielen. Ferner zählen zu Waschmitteln im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

In allen hierin beschriebenen Mitteln kann die Riechstoffzusammensetzung vorzugsweise in den für Duftstoffe/Parfüme üblichen Mengen enthalten sein. In verschiedenen Ausführungsformen sind das, abhängig von der Art des Mittels, Mengen im Bereich von zwischen 0,001 und 99 Gew.-%, vorteilhafterweise zwischen 0,01 und 5 Gew.-%, weiter vorteilhaft zwischen 0,05 und 1 Gew.-%, jeweils bezogen auf das gesamte Mittel.

Neben den beschriebenen Duftstoffen können die Wasch- und Reinigungsmittel selbstverständlich übliche Inhaltsstoffe von solchen Mitteln enthalten. Hier sind in erster Linie Tenside, Buildersubstanzen sowie Bleichmittel, Enzyme und andere Aktivstoffe zu nennen. Zu den wesentlichen Inhaltsstoffen von Wasch- und Reinigungsmitteln gehören dabei insbesondere Tenside.

Je nach Einsatzzweck der erfindungsgemäßen Mittel wird man den Tensidgehalt höher oder niedriger wählen. Üblicherweise liegt der Tensidgehalt von Waschmitteln zwischen 10 und 40 Gew.-%, vorzugsweise zwischen 12,5 und 30 Gew.-% und insbesondere zwischen 15 und 25 Gew.-%, während Reinigungsmittel für das maschinelle Geschirrspülen zwischen 0,1 und 10 Gew.-%, vorzugsweise zwischen 0,5 und 7,5 Gew.-% und insbesondere zwischen 1 und 5 Gew.-% Tenside enthalten.

Diese grenzflächenaktiven Substanzen stammen aus der Gruppe der anionischen, nichtionischen, zwitterionischen oder kationischen Tenside, wobei anionische und nichtionische Tenside aus ökonomischen Gründen und aufgrund ihres Leistungsspektrums beim Waschen und Reinigen deutlich bevorzugt sind.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium-, Kalium- oder Magnesiumsalze, insbesondere in Form der Natriumsalze vor.

Bei der Auswahl der anionischen Tenside stehen der Formulierungsfreiheit keine einzuhaltenden Rahmenbedingungen im Weg. Bevorzugt einzusetzende anionische Tenside sind dabei die Alkylbenzolsulfonate und Fettalkoholsulfate, wobei bevorzugte Waschmittelformkörper 2 bis 20 Gew.-%, vorzugsweise 2,5 bis 15 Gew.-% und insbesondere 5 bis 10 Gew.-% Fettalkoholsulfat(e), jeweils bezogen auf das Gewicht der Mittel, enthalten.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Eine weitere Klasse von nichtionischen Tensiden, die vorteilhaft eingesetzt werden kann, sind die Alkylpolyglycoside (APG). Einsetzbare Alkylpolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglycoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (III), 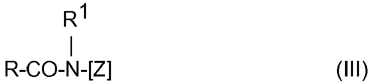 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (IV), 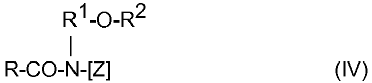 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielsweise nach der Lehre der internationalen Anmeldung WO-A-95/07331 durch Umsetzung mit Fettsäuremethylestern in Gegenwart eines Alkoxids als Katalysator in die gewünschten Polyhydroxyfettsäureamide überführt werden.

Eine andere bedeutende Gruppe von Wasch- und Reinigungsmittelinhaltsstoffen sind die Buildersubstanzen. Unter dieser Substanzklasse, werden sowohl organische als auch anorganische Gerüstsubstanzen verstanden. Es handelt sich dabei um Verbindungen, die sowohl eine Trägerfunktion in den erfindungsgemäßen Mitteln wahrnehmen können als auch bei der Anwendung als wasserenthärtende Substanz wirken.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit, wie beispielsweise in den erfindungsgemäßen Granulaten, auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Diese Substanzklasse wurde im Detail bereits weiter oben beschrieben. Die (co-)polymeren Polycarboxylate können entweder als Pulver oder als wässrige Lösung eingesetzt werden. Der Gehalt der Mittel an (co-)polymeren Polycarboxylaten beträgt vorzugsweise 0,5 bis 20 Gew.-%, insbesondere 3 bis 10 Gew.-%.

Zur Verbesserung der Wasserlöslichkeit können die Polymere auch Allylsulfonsäuren, wie beispielsweise in der EP-B-0 727 448 Allyloxybenzolsulfonsäure und Methallylsulfonsäure, als Monomer enthalten. Insbesondere bevorzugt sind auch biologisch abbaubare Polymere aus mehr als zwei verschiedenen Monomereinheiten, beispielsweise solche, die gemäß der DE-A-43 00 772 als Monomere Salze der Acrylsäure und der Maleinsäure sowie Vinylalkohol bzw. Vinylalkohol-Derivate oder gemäß der DE-C-42 21 381 als Monomere Salze der Acrylsäure und der 2-Alkylallylsulfonsäure sowie Zucker-Derivate enthalten. Weitere bevorzugte Copolymere sind solche, die in den deutschen Patentanmeldungen DE-A-43 03 320 und DE-A-44 17 734 beschrieben werden und als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, von denen in der deutschen Patentanmeldung DE-A-195 40 086 offenbart wird, dass sie neben Cobuilder-Eigenschaften auch eine bleich-stabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, beispielsweise wie in der europäischen Patentanmeldung EP-A-0 280 223 beschrieben, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch so genannte Gelbdextrine und Weißdextrine mit höheren Molmassen im Bereich von 2000 bis 30000 g/mol. Ein bevorzugtes Dextrin ist in der britischen Patentanmeldung 94 19 091 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP-A-0 232 202, EP-A-0 427 349, EP-A-0 472 042 und EP-A-0 542 496 sowie den internationalen Patentanmeldungen WO 92/18542, WO-A-93/08251, WO-A-93/16110, WO-A-94/28030, WO-A-95/07303, WO-A-95/12619 und WO-A-95/20608 bekannt. Ebenfalls geeignet ist ein oxidiertes Oligosaccharid gemäß der deutschen Patentanmeldung DE-A-196 00 018. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS), dessen Synthese beispielsweise in US 3 158 615 beschrieben wird, bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den US-amerikanischen Patentschriften US 4 524 009, US 4 639 325, in der europäischen Patentanmeldung EP-A-0 150 930 und der japanischen Patentanmeldung JP 93/339896 beschrieben werden. Geeignete Einsatzmengen liegen in zeolithhaltigen und/oder silicathaltigen Formulierungen bei 3 bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Cobuilder werden beispielsweise in der internationalen Patentanmeldung WO-A-95/20029 beschrieben.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Cobuilder eingesetzt werden.

Ein bevorzugt eingesetzter anorganischer Builder ist feinkristalliner, synthetischer und gebundenes Wasser enthaltender Zeolith. Der eingesetzte feinkristalline, synthetische und gebundenes Wasser enthaltende Zeolith ist vorzugsweise Zeolith A und/oder P. Geeignet sind jedoch auch Zeolith X sowie Mischungen aus A, X und/oder P, beispielsweise ein Co-Kristallisat aus den Zeolithen A und X. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Messmethode: Coulter Counter) auf und enthalten vorzugsweise 18 bis 22 Gew.-%, insbesondere 20 bis 22 Gew.-% an gebundenem Wasser. In bevorzugten Ausführungsformen sind Zeolithe in Mengen von 10 bis 94,5 Gew.-% in dem Vorgemisch enthalten, wobei es kann besonders bevorzugt ist, wenn Zeolithe in Mengen von 20 bis 70, insbesondere 30 bis 60 Gew.-% enthalten sind.

Geeignete Teilsubstitute für Zeolithe sind Schichtsilicate natürlichen und synthetischen Ursprungs. Derartige Schichtsilicate sind beispielsweise aus den Patentanmeldungen DE-A-23 34 899, EP-A-0 026 529 und DE-A-35 26 405 bekannt. Ihre Verwendbarkeit ist nicht auf eine spezielle Zusammensetzung bzw. Strukturformel beschränkt. Bevorzugt sind hier jedoch Smectite, insbesondere Bentonite. Auch kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind, eigenen sich zur Substitution von Zeolithen oder Phosphaten. Derartige kristalline Schichtsilicate werden beispielsweise in der europäischen Patentanmeldung EP-A-0 164 514 beschrieben. Bevorzugte kristalline Schichtsilicate der angegebenen Formel sind solche, in denen M für Natrium steht und x die Werte 2 oder 3 annimmt. Insbesondere sind sowohl ß- als auch δ-Natriumdisilicate Na₂Si₂O₅·yH₂O bevorzugt.

Zu den bevorzugten Builder-Substanzen gehören auch amorphe Natriumsilicate mit einem Modul Na₂O:SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilicaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silicate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silicatpartikel bei Elektronenbeugungsexperimenten verwaschene oder sogar scharfe Beugungsmaxima liefern. Dies ist so zu interpretieren, dass die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silicate, welche ebenfalls eine Löseverzögerung gegenüber den herkömmlichen Wassergläsern aufweisen, werden beispielsweise in der deutschen Patentanmeldung DE-A-44 00 024 beschrieben. Insbesondere bevorzugt sind verdichtete/kompaktierte amorphe Silicate, compoundierte amorphe Silicate und übertrocknete röntgenamorphe Silicate, wobei insbesondere die übertrockneten Silicate bevorzugt auch als Träger in den erfindungsgemäßen Granulaten vorkommen bzw. als Träger in dem erfindungsgemäßen Verfahren eingesetzt werden.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate. Ihr Gehalt beträgt im Allgemeinen nicht mehr als 25 Gew.-%, vorzugsweise nicht mehr als 20 Gew.-%, jeweils bezogen auf das fertige Mittel. In einigen Fällen hat es sich gezeigt, dass insbesondere Tripolyphosphate schon in geringen Mengen bis maximal 10 Gew.-%, bezogen auf das fertige Mittel, in Kombination mit anderen Buildersubstanzen zu einer synergistischen Verbesserung des Sekundärwaschvermögens führen.

Neben den genannten Bestandteilen können die erfindungsgemäßen Wasch- und Reinigungsmittel zusätzlich einen oder mehrere Stoffe aus den Gruppen der Bleichmittel, Bleichaktivatoren, Enzyme, pH-Stellmittel, Fluoreszenzmittel, Farbstoffe, Schauminhibitoren, Silikonöle, Antiredepositionsmittel, optischen Aufheller, Vergrauungsinhibitoren, Farbübertragungsinhibitoren, Korrosionsinhibitoren und Silberschutzmittel enthalten. Geeignete Mittel sind im Stand der Technik bekannt.

Diese Aufzählung von Wasch- und Reinigungsmittelinhaltsstoffen ist keineswegs abschließend, sondern gibt lediglich die wesentlichsten typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um ein- oder mehrwertige Alkohole mit 1 bis 4 C-Atomen. Bevorzugte Alkohole in solchen Mitteln sind Ethanol, 1,2-Propandiol, Glycerin sowie Gemische aus diesen Alkoholen. In bevorzugten Ausführungsformen enthalten derartige Mittel 2 bis 12 Gew.-% solcher Alkohole.

Grundsätzlich können die Mittel verschiedene Aggregatszustände aufweisen. In einer bevorzugten Ausführungsform handelt es sich bei den Wasch- oder Reinigungsmitteln um flüssige oder gelförmige Mittel, insbesondere um Flüssigwaschmittel oder flüssige Geschirrspülmittel oder Reinigungsgele, wobei es sich insbesondere auch um gelförmige Reinigungsmittel für Spültoiletten handeln kann. Derartige gelförmige Reinigungsmittel für Spültoiletten werden beispielsweise in der deutschen Patentanmeldung DE-A-197 158 72 beschrieben.

Weitere typische Reinigungsmittel, die die erfindungsgemäßen Riechstoffzusammensetzungen enthalten können, sind flüssige oder gelförmige Reiniger für harte Oberflächen, insbesondere so genannte Allzweckreiniger, Glasreiniger, Boden- oder Badezimmerreiniger sowie spezielle Ausführungsformen derartiger Reiniger wozu saure oder alkalische Formen von Allzweckreinigern ebenso wie Glasreiniger mit so genannter Anti-Rain-Wirkung gehören. Dabei können diese flüssigen Reinigungsmittel sowohl in einer als auch in mehreren Phasen vorliegen. In einer insbesondere bevorzugten Ausführungsform weisen die Reiniger 2 verschiedene Phasen auf.

Reiniger ist dabei im weitesten Sinne eine Bezeichnung für - zumeist Tensid-haltige - Formulierungen mit sehr weitem Einsatzbereich und davon abhängig sehr unterschiedlicher Zusammensetzung. Die wichtigsten Marktsegmente sind Haushalts-Reiniger, industrielle (technische) und institutionelle Reiniger. Nach dem pH-Wert unterscheidet man alkalische, neutrale und saure Reiniger, nach der Angebotsform flüssige und feste Reiniger (auch in Tablettenform). Die so genannten Reiniger für harte Oberflächen sollen im Unterschied etwa zu Geschirrspülmitteln, die ebenfalls mit in die Produktgruppe der Reiniger eingeordnet werden, sowohl im konzentrierten Zustand als auch in verdünnter wässriger Lösung. in Verbindung mit mechanischer Energie ein optimales Anwendungsprofil zeigen. Kaltreiniger entfalten ihre Leistung ohne erhöhte Temperatur. Maßgebend für die Reinigungswirkung sind vor allem Tenside und/oder Alkaliträger, alternativ Säuren, ggf. auch Lösungsmittel wie Glykolether und niedere Alkohole. Im allgemeinen sind in den Formulierungen darüber hinaus Builder, je nach Reiniger-Typ auch Bleichmittel, Enzyme, keimmindernde oder desinfizierende Zusätze sowie Parfümöle und Farbstoffe enthalten. Reiniger können auch als Mikroemulsionen formuliert sein. Der Reinigungserfolg hängt in hohem Maße von der - auch geographisch sehr unterschiedlichen - Schmutzart und den Eigenschaften der zu reinigenden Oberflächen ab.

Die Reiniger können als Tensidkomponente anionische, nichtionische, amphotere oder kationische Tenside bzw. Tensidgemische aus einer, mehreren oder allen diesen Tensidklassen enthalten. Die Reiniger enthalten Tenside in Mengen, bezogen auf die Zusammensetzung, von 0,01 bis 30 Gew.-%, vorzugsweise 0,1 bis 20 Gew.-%, insbesondere 1 bis 14 Gew.-%, äußerst bevorzugt 3 bis 10 Gew.-%.

Geeignete Niotenside in derartigen Allzweckreinigern sind beispielsweise C₈-C₁₈-Alkylalkoholpolyglykolether, Alkylpolyglykoside sowie stickstoffhaltige Tenside bzw. Mischungen davon, insbesondere der ersten beiden. Die Mittel enthalten nichtionische Tenside in Mengen, bezogen auf die Zusammensetzung, von 0 bis 30 Gew.-%, vorzugsweise 0,1 bis 20 Gew.-%, insbesondere 0,5 bis 14 Gew.-%, äußerst bevorzugt 1 bis 10 Gew.-%.

C₈₋₁₈-Alkylalkoholpolypropylenglykol/polyethylenglykolether stellen bekannte nichtionische Tenside dar. Sie können durch die Formel RⁱO-(CH₂CH(CH₃)O)*ₚ*(CH₂CH₂O)*ₑ*-H, beschrieben werden, in der Rⁱ für einen linearen oder verzweigten, aliphatischen Alkyl- und/oder Alkenylrest mit 8 bis 18 Kohlenstoffatomen, p für 0 oder Zahlen von 1 bis 3 und e für Zahlen von 1 bis 20 steht. Die C₈₋₁₈-Alkylalkoholpolyglykolether kann man durch Anlagerung von Propylenoxid und/oder Ethylenoxid an Alkylalkohole, vorzugsweise an Fettalkohole, erhalten. Typische Beispiele sind Polyglykolether in der Rⁱ für einen Alkylrest mit 8 bis 18 Kohlenstoffatomen, p für 0 bis 2 und e für Zahlen von 2 bis 7 steht. Bevorzugte Vertreter sind beispielsweise C₁₀-C₁₄-Fettalkohol+1PO+6EO-ether (*p* = 1, *e* = 6) und C₁₂-C₁₈-Fettalkohol+7EO-ether (*p* = 0, *e* = 7) sowie deren Mischungen.

Es können auch endgruppenverschlossene C₈-C₁₈-Alkylalkoholpolyglykolether eingesetzt werden, d.h. Verbindungen in denen die freie OH-Gruppe verethert ist. Die endgruppenverschlossenen C₈₋₁₈-Alkylalkoholpolyglykolether können nach einschlägigen Methoden der präparativen organischen Chemie erhalten werden. Vorzugsweise werden C₈₋₁₈-Alkylalkohopolyglykolether in Gegenwart von Basen mit Alkylhalogeniden, insbesondere Butyl- oder Benzylchlorid, umgesetzt. Typische Beispiele sind Mischether bei denen Rⁱ für einen technischen Fettalkoholrest, vorzugsweise C_{12/14}-Kokosalkylrest, p für 0 und e für 5 bis 10 stehen, die mit einer Butylgruppe verschlossen sind.

Bevorzugte nichtionische Tenside sind weiterhin die bereits weiter oben beschriebenen Alkylpolyglykoside.

Als weitere nichtionische Tenside können stickstoff-enthaltende Tenside enthalten sein, z.B. Fettsäurepolyhydroxyamide, beispielsweise Glucamide, und Ethoxylate von Alkylaminen, vicinalen Diolen und/oder Carbonsäureamiden, die Alkylgruppen mit 10 bis 22 C-Atomen, vorzugsweise 12 bis 18 C-Atomen, besitzen. Der Ethoxylierungsgrad dieser Verbindungen liegt dabei in der Regel zwischen 1 und 20, vorzugsweise zwischen 3 und 10. Bevorzugt sind Ethanolamid-Derivate von Alkansäuren mit 8 bis 22 C-Atomen, vorzugsweise 12 bis 16 C-Atomen. Zu den besonders geeigneten Verbindungen gehören die Laurinsäure-, Myristinsäure- und Palmitinsäuremonoethanolamide.

Für Allzweckreiniger geeignete Aniontenside sind C₈₋₁₈-Alkylsulfate, C₈₋₁₈-Alkylethersulfate, d.h. die Sulfatierungsprodukte von Alkoholethern und/oder C₈₋₁₈-Alkylbenzolsulfonate, aber auch C₈₋₁₈-Alkansulfonate, C₈₋₁₈-α-Olefinsulfonate,sulfonierte C₈₋₁₈-Fettsäuren, insbesondere Dodecylbenzolsulfonat, C₈₋₂₂-Carbonsäureamidethersulfate, Sulfonbernsteinsäuremono- und -di-C₁₋₁₂-Alkylester, C₈₋₁₈-Alkylpolyglykolethercarboxylate, C₈₋₁₈-N-Acyltauride, C₈₋₁₈-N-Sarkosinate und C₈₋₁₈-Alkylisethionate bzw. deren Mischungen. Sie werden in Form ihrer Alkalimetall- und Erdalkalimetallsalze, insbesondere Natrium-, Kalium- und Magnesiumsalze, wie auch Ammonium- und Mono-, Di-, Tri- bzw. Tetraalkylammoniumsalze sowie im Falle der Sulfonate auch in Form ihrer korrespondierende Säure, z.B. Dodecylbenzolsulfonsäure, eingesetzt. Die Mittel enthalten anionische Tenside in Mengen, bezogen auf die Zusammensetzung, von 0 bis 30 Gew.-%, vorzugsweise 0,1 bis 20 Gew.-%, insbesondere 1 bis 14 Gew.-%, äußerst bevorzugt 2 bis 10 Gew.-%.

Wegen ihrer schaumdämpfenden Eigenschaften können die Allzweckreiniger auch Seifen, d.h. Alkali- oder Ammoniumsalze gesättigter oder ungesättigter C₆₋₂₂-Fettsäuren, enthalten. Die Seifen können in einer Menge bis zu 5 Gew.-%, vorzugsweise von 0,1 bis 2 Gew.-%, eingesetzt werden.

Geeignete Amphotenside sind beispielsweise Betaine der Formel (Rⁱⁱ)(Rⁱⁱⁱ)(R^{iv})N⁺CH₂COO⁻, in der Rⁱⁱ einen gegebenenfalls durch Heteroatome oder Heteroatomgruppen unterbrochenen Alkylrest mit 8 bis 25, vorzugsweise 10 bis 21 Kohlenstoffatomen und Rⁱⁱⁱ sowie R^{iv} gleichartige oder verschiedene Alkylreste mit 1 bis 3 Kohlenstoffatomen bedeuten, insbesondere C₁₀₋₁₈-Alkyl-dimethylcarboxymethylbetain und C₁₁₋₁₇-Alkylamidopropyl-dimethylcarboxymethylbetain. Die Mittel enthalten amphotere Tenside in Mengen, bezogen auf die Zusammensetzung, von 0 bis 15 Gew.-%, vorzugsweise 0,01 bis 10 Gew.-%, insbesondere 0,1 bis 5 Gew.-%.

Geeignete Kationtenside sind u.a. die quartären Ammoniumverbindungen der Formel (R^{v})(R^{vi})(R^{vii})(R^{viii})N⁺ X⁻, in der R^{v} bis R^{viii} für vier gleich- oder verschiedenartige, insbesondere zwei lang- und zwei kurzkettige, Alkylreste und X⁻ für ein Anion, insbesondere ein Halogenidion, stehen, beispielsweise Didecyl-dimethyl-ammoniumchlorid, Alkyl-benzyl-didecyl-ammoniumchlorid und deren Mischungen. Die Mittel enthalten kationische Tenside in Mengen, bezogen auf die Zusammensetzung, von 0 bis 10 Gew.-%, vorzugsweise 0,01 bis 5 Gew.-%, insbesondere 0,1 bis 3 Gew.-%.

In einer bevorzugten Ausführungsform enthalten die Reiniger anionische und nichtionische Tenside nebeneinander, vorzugsweise C₈₋₁₈-Alkylbenzolsulfonate, C₈₋₁₈-Alkylsulfate und/oder C₈₋₁₈-Alkylethersulfate neben C₈₋₁₈-Alkylalkoholpolyglykolethern und/oder Alkylpolyglykosiden, insbesondere C₈₋₁₈-Alkylbenzolsulfonate neben C₈₋₁₈-Alkylalkoholpoly-glykolethern.

Weiterhin können die erfindungsgemäßen Reiniger Builder enthalten. Geeignete Builder sind beispielsweise Alkalimetallgluconate, -citrate, -nitrilotriacetate, -carbonate und -bicarbonate, insbesondere Natriumgluconat, -citrat und -nitrilotriacetat sowie Natrium- und Kaliumcarbonat und -bicarbonat, sowie Alkalimetall- und Erdalkalimetallhydroxide, insbesondere Natrium- und Kaliumhydroxid, Ammoniak und Amine, insbesondere Mono- und Triethanolamin, bzw. deren Mischungen. Hierzu zählen auch die Salze der Glutarsäure, Bernsteinsäure, Adipinsäure, Weinsäure und Benzolhexacarbonsäure sowie Phosphonate und Phosphate. Die Mittel enthalten Builder in Mengen, bezogen auf die Zusammensetzung, von 0 bis 20 Gew.-%, vorzugsweise 0,01 bis 12 Gew.-%, insbesondere 0,1 bis 8 Gew.-%, äußerst bevorzugt 0,3 bis 5 Gew.-%, wobei jedoch die Menge an Natriumhexametaphosphat - ausgenommen die verwendungsgemäßen Mittel - auf 0 bis 5 Gew.-% beschränkt ist. Als Elektrolyte sind die Buildersalze zugleich Phasentrennhilfsmittel.

Neben den genannten Komponenten können die erfindungsgemäßen Reiniger weitere Hilfs- und Zusatzstoffe enthalten, wie sie in derartigen Mitteln üblich sind. Hierzu zählen insbesondere Polymere, Soil-Release-Wirkstoffe, Lösungsmittel (z.B. Ethanol, Isopropanol, Glykolether), Lösungsvermittler, Hydrotrope (z.B. Cumolsulfonat, Octylsulfat, Butylglucosid, Butylglykol), Reinigungsverstärker, Viskositätsregler (z.B. synthetische Polymere wie Polysaccharide, Polyacrylate, in der Natur vorkommenden Polymere und deren Derivate wie Xanthangum, weitere Polysaccharide und/oder Gelatine), pH-Regulatoren (z.B. Citronensäure, Alkanolamine oder NaOH), Desinfektionsmittel, Antistatika, Konservierungsmittel, Bleichsysteme, Enzyme, Farbstoffe sowie Trübungsmittel oder auch Hautschutzmittel, wie sie in EP-A-0 522 506 beschrieben sind. Die Menge an derartigen Zusätzen liegt üblicherweise nicht über 12 Gew.-% im Reinigungsmittel. Die Untergrenze des Einsatzes hängt von der Art des Zusatzstoffes ab und kann beispielsweise bei Farbstoffen bis zu 0,001 Gew.-% und darunter betragen. Vorzugsweise liegt die Menge an Hilfsstoffen zwischen 0,01 und 7 Gew.-%, insbesondere 0,1 und 4 Gew.-%.

Der pH-Wert der Allzweckreiniger kann über einen weiten Bereich variiert werden, bevorzugt ist jedoch ein Bereich von 2,5 bis 12, insbesondere 5 bis 10,5. Unter dem pH-Wert ist dabei der vorliegenden Erfindung der pH-Wert des Mittels in Form der temporären Emulsion zu verstehen.

Derartige Allzweckreiniger-Rezepturen lassen sich für beliebige Zwecke modifizieren. Eine besondere Ausführungsform sind dabei die Glasreiniger. Wesentlich bei derartigen Reinigern ist, dass Flecken oder Ränder zurückbleiben. Insbesondere ist hierbei ein Problem, dass nach dem Reinigen Wasser auf diesen Oberflächen kondensiert und zu dem sogenannten Beschlageffekt führt. Ebenso ist es unerwünscht, wenn auf Glasscheiben die dem Regen ausgesetzt sind, sogenannte Regenflecken zurückbleiben. Dieser Effekt ist als Regeneffekt oder Anti-Rain-Effekt bekannt. Diesen Effekten kann durch geeignete Additive in Glasreinigern vorgebeugt werden.

In einer anderen bevorzugten Ausführungsform handelt es sich bei den Mitteln um pulverförmige oder granulatförmige Mittel. Die erfindungsgemäßen Mittel können dabei beliebige Schüttgewichte aufweisen. Die Palette der möglichen Schüttgewichte reicht von niedrigen Schüttgewichten unter 600 g/l, beispielsweise 300 g/l, über den Bereich mittlerer Schüttgewichte von 600 bis 750 g/l bis zum Bereich hoher Schüttgewichte von mindestens 750 g/l.

Zur Herstellung solcher Mittel sind beliebige, aus dem Stand der Technik bekannte Verfahren, geeignet.

Ebenfalls Gegenstand der Erfindung sind Luftpflegemittel, die die Riechstoffzusammensetzungen der Erfindung enthalten. Das Luftpflegemittel kann z.B. ein Raumlufterfrischer, Raumdeodorant oder Raumspray sein.

Ein weiterer Gegenstand der vorliegenden Erfindung sind kosmetische Mittel, beispielsweise zur Haar- oder Hautbehandlung, die die hierin beschriebenen Riechstoffzusammensetzungen vorzugsweise in den bereits oben im Zusammenhang mit den anderen Mitteln beschriebenen Mengen enthalten. In einer bevorzugten Ausführungsform handelt es sich bei den kosmetischen Mitteln um wässrige Zubereitungen, die oberflächenaktive Wirkstoffe enthalten und die sich insbesondere zur Behandlung von Keratinfasern, insbesondere menschlichen Haaren, oder zur Behandlung von Haut eignen.

Bei den angesprochenen Haarbehandlungsmitteln handelt es sich dabei insbesondere um Mittel zur Behandlung von menschlichem Kopfhaar. Die gebräuchlichsten Mittel dieser Kategorie lassen sich einteilen in Haarwaschmittel, Haarpflegemittel, Haarverfestigungs- und Haarverformungsmittel sowie Haarfärbemittel und Haarentfernungsmittel. Zu den erfindungsgemäß bevorzugten, oberflächenaktive Wirkstoffe enthaltenden Mittel zählen insbesondere Haarwasch- und Pflegemittel. Diese wässrigen Zubereitungen liegen meist in flüssig bis pastöser Form vor.

Für die wichtigste Inhaltsstoff-Gruppe, die oberflächenaktiven Wirkstoffe oder Waschaktivstoffe, werden überwiegend Fettalkoholpolyglykolethersulfate (Ethersulfate, Alkylethersulfate) eingesetzt, zum Teil in Kombination mit anderen meist anionischen Tensiden. Shampoo-Tenside sollen außer guter Reinigungskraft und Unempfindlichkeit gegen Wasserhärte Haut- und Schleimhautverträglichkeit aufweisen. Entsprechend den gesetzlichen Regelungen muss gute biologische Abbaubarkeit gegeben sein. Neben den Alkylethersulfaten können bevorzugte Mittel zusätzlich weitere Tenside, wie Alkylsulfate, Alkylethercarboxylate, vorzugsweise mit Ethoxylierungsgraden von 4 bis 10, sowie tensidische Eiweiß-Fettsäure-Kondensate enthalten.

Haarpflegemittel haben zum Ziel den Naturzustand des frisch nachgewachsenen Haares möglichst lange zu erhalten und bei Schädigung wiederherzustellen. Merkmale die diesen Naturzustand charakterisieren sind seidiger Glanz, geringe Porosität, spannkräftige und dabei weiche Fülle und angenehm glattes Gefühl. Eine wichtige Voraussetzung hierfür ist eine saubere, schuppenfreie und nicht überfettete Kopfhaut. Zu den Haarpflegemitteln zählt man heute eine Vielzahl verschiedener Produkte, deren wichtigste Vertreter als Vorbehandlungsmittel, Haarwässer, Frisierhilfsmittel, Haarspülungen und Kurpackungen bezeichnet werden.

Bei den wässrigen Zubereitungen zur Behandlung von Haut handelt es sich insbesondere um Zubereitungen zur Pflege menschlicher Haut. Diese Pflege beginnt mit der Reinigung für die in erster Linie Seifen benutzt werden. Hier unterscheidet man feste, meist stückige und flüssige Seife. Dementsprechend liegen die kosmetischen Mittel in einer bevorzugten Ausführungsform als Formkörper vor, die oberflächenaktive Inhaltsstoffe enthalten. Wichtigste Inhaltsstoffe derartiger Formkörper sind in einer bevorzugten Ausführungsform die Alkalisalze der Fettsäuren natürlicher Öle und Fette, vorzugsweise mit Ketten von 12-18 C-Atomen. Da Laurinsäure-Seifen besonders gut schäumen, sind die Laurinsäure-reichen Kokos- und Palmkern-Öle bevorzugte Rohstoffe für die Feinseifen-Herstellung. Die Na-Salze der Fettsäure-Gemische sind fest, die K-Salze weichpastös. Zur Verseifung wird die verdünnte Natron- oder Kali-Lauge den Fett-Rohstoffen im stöchiometrischem Verhältnis so zugesetzt, dass in der fertigen Seife ein Laugenüberschuss von max. 0,05% vorhanden ist. Vielfach werden die Seifen heute nicht mehr direkt aus den Fetten, sondern aus den durch Fettspaltung gewonnenen Fettsäuren hergestellt. Übliche Seifen-Zusätze sind Fettsäuren, Fettalkohole, Lanolin, Lecithin, pflanzliche Öle, Partialglyceride u.a. fettähnliche Substanzen zur Rückfettung der gereinigten Haut, Antioxidantien wie Ascorbyl-Palmitat oder Tocopherol zur Verhinderung der Autooxidation der Seife (Ranzigkeit), Komplexierungsmittel wie Nitrilotriacetat zur Bindung von Schwermetall-Spuren, die den autooxidativen Verderb katalysieren könnten, Parfüm-Öle zur Erzielung der gewünschten Duftnoten, Farbstoffe zur Einfärbung der Seifenstücke und ggf. spezielle Zusätze.

Flüssige Seifen basieren sowohl auf K-Salzen natürlicher Fettsäuren als auch auf synthetischen Aniontensiden. Sie enthalten in wässriger. Lösung. weniger waschaktive Substanzen als feste Seifen, haben die üblichen Zusätze, ggf. mit viskositätsregulierenden Bestandteilen sowie Perlglanz-Additiven. Wegen ihrer bequemen und hygienischen Anwendung aus Spendern werden sie vorzugsweise in öffentlichen Waschräumen und dergleichen verwendet. Wasch-Lotionen für besonders empfindliche Haut basieren auf mild wirkenden synthetischen Tensiden mit Zusätzen hautpflegender Substanzen, pH-neutral oder schwach sauer (pH 5,5) eingestellt.

Zur Reinigung vornehmlich der Gesichtshaut gibt es eine Reihe weitere Präparate, wie Gesichtswässer, Reinigungs-Lotionen, -Milche, -Cremes, -Pasten; Gesichtspackungen dienen z.T. der Reinigung, überwiegend jedoch der Erfrischung und Pflege der Gesichtshaut. Gesichtswässer sind meist wässrige-alkoholische Lösungen mit geringen Tensid-Anteilen sowie weiteren hautpflegenden Substanzen. Reinigungs-Lotionen, -Milche, -Cremes und -Pasten basieren meist auf O/W-Emulsionen mit relativ geringen Gehalten an Fettkomponenten mit reinigenden und pflegenden Zusätzen. Sogenannte Scruffing- und Peeling-Präparate enthalten mild keratolytisch wirkende Substanzen zur Entfernung der obersten abgestorbenen Haut-Horn-Schichten, z.T. mit Zusätzen abrasiv wirkender Pulver. Die seit langem als mildes Hautreinigungsmittel verwendete Mandelkleie ist auch heute noch vielfach Bestandteil solcher Präparate. In Mitteln zur reinigenden Behandlung unreiner Haut sind außerdem antibakterielle und entzündungshemmende Substanzen enthalten, da die Talgansammlungen in Komedonen (Mitessern) Nährböden für bakterielle Infektionen darstellen und zu Entzündungen neigen. Die angebotene breite Palette verschiedener Hautreinigungs-Produkte variiert in Zusammensetzung und Gehalt an diversen Wirkstoffen, abgestimmt auf die verschiedenen Hauttypen und auf spezielle Behandlungsziele.

Weitere erfindungsgemäß bevorzugte kosmetische Mittel sind Mittel zur Beeinflussung des Körpergeruchs. Insbesondere sind hier deodorierende Mittel gemeint. Derartige Deodorantien können Gerüche überdecken, entfernen oder zerstören. Unangenehme Körpergerüche entstehen bei bakterieller Zersetzung des Schweißes, insbesondere in den feuchtwarmen Achselhöhlen, wo Mikroorganismen gute Lebensbedingungen finden. Dementsprechend sind die wichtigsten Inhaltsstoffe von Deodorantien keimhemmende Substanzen. Insbesondere sind solche keimhemmenden Substanzen bevorzugt, die eine weitgehende selektive Wirksamkeit gegenüber den für den Körpergeruch verantwortlichen Bakterien besitzen. Bevorzugte Wirkstoffe haben dabei jedoch lediglich eine bakteriostatische Wirkung und töten die Bakterienflora keinesfalls ganz ab. Zu dem keimhemmenden Mitteln können generell alle geeigneten Konservierungsmittel mit spezifischer Wirkung gegen grampositiver Bakterien gerichtet werden. Beispielsweise sind dies Irgasan DP 300 (Trichlosan, 2,4,4'-Trichlor-2'-Hydroxydiphenylether), Chlorhexidin (1,1'-Hexamethylenbis(5-(4'-chlor-phenyl)-biguanid) sowie 3,4,4`-Trichlorcarbanilid. Auch quartäre Ammonium-Verbindungen sind prinzipiell ebenfalls geeignet. Aufgrund ihrer hohen antimikrobierenden Wirksamkeit werden all diese Stoffe bevorzugt nur in geringen Konzentrationen von etwa 0,1 bis 0,3 Gew.-% eingesetzt. Weiterhin haben auch zahlreiche Riechstoffe antimikrobielle Eigenschaften. Dementsprechend werden derartige Riechstoffe mit antimikrobiellen Eigenschaften in Deodorantien bevorzugt eingesetzt. Insbesondere sind hier Farnesol und Phenoxyethanol zu nennen. Daher ist es insbesondere bevorzugt, wenn die erfindungsgemäßen Deodorantien solche selbst bakteriostatisch wirksamen Riechstoffe enthalten. Dabei können die Riechstoffe vorzugsweise wieder in Form der hierin beschriebenen Riechstoffzusammensetzungen enthalten sein. Eine weitere Gruppe wesentlicher Inhaltsstoffe von Deodorantien sind Enzyminhibitoren, die die Zersetzung des Schweißes durch Enzyme hemmen, wie beispielsweise Citronensäuretriethylester oder Zinkglycinat. Wesentliche Inhaltsstoffe von Deodorantien sind weiterhin auch Antioxidantien, die eine Oxidation der Schweißbestandteile verhindern sollen.

Bei dem kosmetischen Mittel kann es sich auch um ein Haarfestlegemittel, das zur Festigung Polymere enthält. Besonders bevorzugt ist es dabei, wenn unter den Polymeren mindestens ein Polyurethan enthalten ist. Dabei kann die Zugabe des Mittels, dass die Riechstoffzusammensetzung nach der Erfindung enthält, durch Sprühen erfolgt. Dabei kann die Zugabe durch eine Spraydose (Druckgasdose, Druckgaspackung, Aerosolpackung) oder einem mechanisch zu bedienendem Pumpzerstäuber (Pumpspray) unter Bildung eines Sprühnebels, Schaums, Paste oder Flüssigkeitsstrahls erfolgen.

Die erfindungsgemäße Riechstoffzusammensetzung kann auch in Klebstoffen enthalten sein. Solche Klebstoffe können, ohne darauf beschränkt zu sein, Schmelz-, lösemittelhaltige Nass, Kontakt-, Dispersions-, wasserbasierte Klebstoffe, Plastisole sowie Polymerisations-, Polykondensations-, Polyadditions- und Haftklebstoffe sein.

Ein weiterer Gegenstand der Erfindung betrifft die Verwendung der hierin beschriebenen Wasch-Reinigungs- oder Pflegemittel zum Reinigen und/oder Konditionieren von textilen Flächengebilden. Hierbei wird das zu reinigende Textil einer Textilwäsche unter Einsatz eines erfindungsgemäßen Wasch-, Reinigungs- oder Pflegemittels unterworfen wird, insbesondere in einer automatischen Waschmaschine, vorzugsweise bei Temperaturen nicht über 60 °C, insbesondere nicht über 40 °C.

Ebenso können die erfindungsgemäßen Wasch-, Reinigungs- oder Pflegemittel zum Reinigen harter Oberflächen (z.B. Geschirr, Fußboden, WC) verwendet werden.

Grundsätzlich sind alle im Zusammenhang mit den Riechstoffzusammensetzungen sowie den Mitteln der Erfindung offenbarten Ausführungsformen auch auf die beschriebenen Verfahren und Verwendungen anwendbar und umgekehrt. So ist es beispielsweise selbstverständlich, dass alle hierin beschriebenen speziellen Riechstoffzusammensetzungen in den genannten Mitteln und Verfahren anwendbar sind und wie hierin beschrieben verwendet werden können.

### Beispiele

### Beispiel 1: Herstellung einer Riechstoffzusammensetzung

Damascone wurde in äquimolarer Menge zum Thiol vorlegt und das Thiol (p-Menth-1-en-8-thiol) portionsweise zugegeben. Das Gemisch wurde bis zur vollständigen Homogenisierung gerührt und dann in handelübliche Produkte (ein Universalwaschpulver, ein Flüssigwaschmittel und einen Weichspüler) in unten vorgegebener Menge eingearbeitet.

Die Produkte wurden dann im Folgenden mit verschiedenen Textilien und nach verschiedenen Schritten des Waschvorgangs getestet:

**Waschbedingungen**

| | |
|---|---|
| Waschmaschine | Miele Softtronic W1734 |
| Programm | Hauptwaschgang bei 40 °C mit |
| | a) Universalwaschmittelpulver (unparfürmiert) oder |
| | b) Flüssigwaschmittel (unparfürmiert) |
| Wasserhärte: | 16°dH |
| Schleuderzahl: | 1200U/min |
| Menge Textilien: | 3 kg (Baumwollfrottee & Halbleinen) |
| Prüftextilien: | Baumwollfrottee 30x30cm |

**Bedingungen Trocknung**

| | |
|---|---|
| Temperatur: | 20 °C |
| Luftfeuchtigkeit: | 60 % rh |
| Beurteilung: | Frisch, nach 1 Tag und nach 4 Tagen |
| Probanden: | 10 |
| Reproduktion: | 2 |

**Spülbedingungen**

| | |
|---|---|
| Waschmaschine | Miele Softtronic W1734 |
| Programm | Spülgang mit konz. Weichspüler (unparfümiert) |
| Wasserhärte: | 16 °dH |
| Schleuderzahl: | 1200U/min |
| Menge Textilien: | 3 kg (Baumwollfrottee & Halbleinen) |
| Prüftextilien: | Baumwollfrottee 30x30cm |

**Bedingungen Trocknung**

| | |
|---|---|
| Temperatur: | 20 °C |
| Luftfeuchtigkeit: | 60 % rh |
| Beurteilung: | Frisch, nach 1 Tag und nach 4 Tagen |
| Probanden: | 10 |
| Reproduktion: | 2 |

Die Ergebnisse dieser Versuche sind in den folgenden Tabellen 1-3 dargestellt. Beurteilt wurde jeweils die Duft-Intensität durch Probanden auf einer Skala von 1 bis 10, wobei 1 sehr schwach und 10 sehr stark bedeutet. Die angegebene Dosierung bezieht sich auf die Menge der Riechstoffzusammensetzung bezogen auf das Gesamtgewicht des Mittels

**Tabelle1: Universalwaschmittelpulver**

| Riechstoff | Dosierung | Textil | Produkt | Feucht | Trocken | TW nach 7 Tagen |
|---|---|---|---|---|---|---|
| Thiol | 0,20% | Baumwolle | 4,40 | 3,40 | 1,20 | 1,15 |
| | | Halbleinen | - | 2,70 | 2,20 | 1,25 |
| | | Polyester | - | 2,80 | 2,50 | 1,38 |
| Damascone + Thiol | 0,38% | Baumwolle | 4,60 | 4,40 | 3,20 | 2,50 |
| | | Halbleinen | - | 2,60 | 2,50 | 2,25 |
| | | Polyester | - | 2,70 | 3,00 | 2,75 |

**Tabelle 2: Flüssigwaschmittel**

| Riechstoff | Dosierung | Textil | Produkt | Feucht | Trocken | TW nach 7 Tagen |
|---|---|---|---|---|---|---|
| Thiol | 0,35% | Baumwolle | 3,80 | 3,00 | 1,00 | 1,63 |
| | | Halbleinen | - | 2,40 | 1,20 | 1,25 |
| | | Polyester | - | 2,40 | 2,30 | 1,88 |
| Damascone + Thiol | 0,66% | Baumwolle | 4,80 | 3,80 | 3,60 | 3,00 |
| | | Halbleinen | - | 3,80 | 2,40 | 2,00 |
| | | Polyester | - | 3,80 | 3,60 | 2,75 |

**Tabelle 3: Weichspüler**

| Riechstoff | Dosierung | Textil | Produkt | Feucht | Trocken |
|---|---|---|---|---|---|
| Thiol | 0,45% | Baumwolle | 4,40 | 4,40 | 2,40 |
| | | Halbleinen | - | 4,40 | 1,80 |
| | | Polyester | - | 3,80 | 3,40 |
| Damascone + Thiol | 0,85% | Baumwolle | 4,80 | 5,00 | 3,80 |
| | | Halbleinen | - | 4,40 | 3,40 |
| | | Polyester | - | 4,80 | 3,40 |

## Patentansprüche

1. Riechstoffzusammensetzung, **dadurch gekennzeichnet, dass** die Riechstoffzusammensetzung enthält:
(i) mindestens eine α,β-ungesättigte Verbindung ausgewählt aus α,β-ungesättigten Ketonen, α,β-ungesättigten Aldehyden oder aus Mischungen daraus; und
(ii) mindestens einen Thiolgruppen-haltigen Riechstoff.
wobei die mindestens eine α,β-ungesättigte Verbindung (i) und der mindestens eine Thiolgruppen-haltige Riechstoff (ii) im molaren Verhältnis zwischen 5:1 und 1:1 eingesetzt werden.

2. Riechstoffzusammensetzung nach Anspruch 1, **dadurch gekennzeichnet, dass** die mindestens eine α,β-ungesättigte Verbindung ein Riechstoff ist.

3. Riechstoffzusammensetzung nach Anspruch 1 oder 2, **dadurch gekennzeichnet, dass** die mindestens eine α,β-ungesättigte Verbindung eine Verbindung nach Formel (I) ist
R¹-CH=CH-C(=O)R² (I)
wobei R¹ und R² jeweils unabhängig voneinander für Wasserstoff, einen substituierten oder unsubstituierten Alkyl-, Cycloalkyl-, Alkenyl-, Cycloalkenyl-, Aryl- oder Acylgruppen-haltigen Rest oder ein Polymer stehen.

4. Riechstoffzusammensetzung nach Anspruch 3, **dadurch gekennzeichnet, dass**
(1) R¹ für einen substituierten oder unsubstituierten Alkylrest mit 1 bis 10 Kohlenstoffatomen steht, insbesondere für einen Methylrest, und/oder
(2) R² für einen Alkyl-, Cycloalkyl-, Alkenyl-, Cycloalkenyl-, Aryl-, Acylgruppe-haltigen Rest mit wenigstens 5 Kohlenstoffatomen steht, insbesondere einen cyclischen, substituierten Alkenyl-Rest mit wenigstens 5 Kohlenstoffatomen umfasst, vorzugsweise

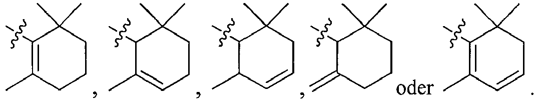

5. Riechstoffzusammensetzung nach Anspruch 4, **dadurch gekennzeichnet, dass** die Verbindung der Formel (I) ausgewählt wird aus mindestens einem Vertreter der Gruppe die gebildet wird aus α-Damascon, β-Damascon, δ-Damascon, γ-Damascon, Damascenon, Zimtaldehyd, Hydroxyzimtaldehyd, Iron, Citral, Janonen, α-Isomethylionon, Methylcedrylketon.

6. Riechstoffzusammensetzung nach einem der Ansprüche 1 bis 5, wobei der Thiolgruppen-haltige Riechstoff ein Riechstoff mit 5-20 Kohlenstoffatomen ist, bevorzugt ausgewählt aus (*R*)-, (*S*)-Thioterpineol (p-Menth-1-en-8-thiol), 2-(1-Mercapto-1-methylethyl)-5-methylcyclohexanon, 4-Methoxy-2-methyl-2-butanthiol, 3-mercapto-1-hexanol, 2-Mercaptopropansäure, 2-Mercaptopropansäureethylester und 3-(Methylthio)propylmercaptoacetat.

7. Mittel, **dadurch gekennzeichnet, dass** das Mittel mindestens eine Riechstoffzusammensetzung nach einem der Ansprüche 1 bis 6 enthält, vorzugsweise in einer Gesamtmenge von 0,001-99 Gew-.%, besonders bevorzugt von 0,05-1 Gew.-%, bezogen auf das Gewicht des Mittels oder des Klebstoffs, wobei das Mittel ein Wasch- oder Reinigungsmittel, Luftpflegemittel, kosmetisches Mittel oder ein Klebstoff ist.

8. Verwendung eines Wasch- oder Reinigungsmittels enthaltend eine Riechstoffzusammensetzung nach einem der Ansprüche 1 bis 6 zum Reinigen und/oder Konditionieren von textilen Flächengebilden, insbesondere in einer automatischen Waschmaschine.

9. Verwendung einer α,β-ungesättigten Verbindung ausgewählt aus α,β-ungesättigten Ketonen, α,β-ungesättigten Aldehyden oder Mischungen daraus zur Stabilisierung eines Thiolgruppen-haltigen Riechstoffs.

10. Verwendung nach Anspruch 9, **dadurch gekennzeichnet, dass** die mindestens eine α,β-ungesättigte Verbindung ein Riechstoff ist.

11. Verwendung nach Anspruch 9 oder 10, **dadurch gekennzeichnet, dass** die mindestens eine α,β-ungesättigte Verbindung eine Verbindung nach Formel (I) ist
R¹-CH=CH-C(=O)R² (I)
wobei R¹ und R² jeweils unabhängig voneinander für Wasserstoff, einen substituierten oder unsubstituierten Alkyl-, Cycloalkyl-, Alkenyl-, Cycloalkenyl-, Aryl- oder Acylgruppen-haltigen Rest oder ein Polymer stehen.

12. Verwendung nach Anspruch 11, **dadurch gekennzeichnet, dass**
(1) R¹ für einen substituierten oder unsubstituierten Alkylrest mit 1 bis 10 Kohlenstoffatomen steht, insbesondere für einen Methylrest, und/oder
(2) R² für einen Alkyl-, Cycloalkyl-, Alkenyl-, Cycloalkenyl-, Aryl-, Acylgruppe-haltigen Rest mit wenigstens 5 Kohlenstoffatomen steht, insbesondere einen cyclischen, substituierten Alkenyl-Rest mit wenigstens 5 Kohlenstoffatomen umfasst, vorzugsweise

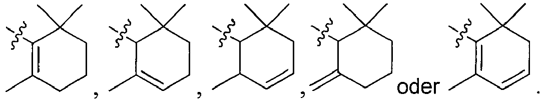

13. Verwendung nach nach Anspruch 12, **dadurch gekennzeichnet, dass** die Verbindung der Formel (I) ausgewählt wird aus mindestens einem Vertreter der Gruppe die gebildet wird aus α-Damascon, β-Damascon, δ-Damascon, γ-Damascon, Damascenon, Zimtaldehyd, Hydroxyzimtaldehyd, Iron, Citral, Janonen, α-Isomethylionon, Methylcedrylketon.

14. Verwendung nach einem der Ansprüche 9 bis 13, wobei der Thiolgruppen-haltige Riechstoff ein Riechstoff mit 5-20 Kohlenstoffatomen ist, bevorzugt ausgewählt aus (*R*)-, (*S*)-Thioterpineol (p-Menth-1-en-8-thiol), 2-(1-Mercapto-1-methylethyl)-5-methyl-cyclohexanon, 4-Methoxy-2-methyl-2-butanthiol, 3-mercapto-1-hexanol, 2-Mercaptopropansäure, 2-Mercaptopropansäureethylester und 3-(Methylthio)propylmercaptoacetat.

## Claims

1. An odorant composition, **characterized in that** the odorant composition contains:
(i) at least one α,β-unsaturated compound selected from α,β-unsaturated ketones, α,β-unsaturated aldehydes or mixtures thereof; and
(ii) at least one odorant that contains thiol groups, wherein
the at least one α,β-unsaturated compound (i) and the at least one odorant (ii) that contains thiol groups being used in a molar ratio of between 5:1 and 1:1.

2. The odorant composition according to claim 1, **characterized in that** the at least one α,β-unsaturated compound is an odorant.

3. The odorant composition according to claim 1 or 2, **characterized in that** the at least one α,β-unsaturated compound is a compound according to formula (I)
R¹-CH=CH-C(=O)R² (I)
where R¹ and R² each represent, independently of one another, hydrogen, a substituted or unsubstituted group containing alkyl, cycloalkyl, alkenyl, cycloalkenyl, aryl or acyl groups, or a polymer.

4. The odorant composition according to claim 3, **characterized in that**
(1) R¹ represents a substituted or unsubstituted alkyl group having from 1 to 10 carbon atoms, in particular a methyl group, and/or
(2) R² represents a group that contains an alkyl, cycloalkyl, alkenyl, cycloalkenyl, aryl or acyl group and has at least 5 carbon atoms, and in particular it comprises a cyclic, substituted alkenyl group having at least 5 carbon atoms, preferably

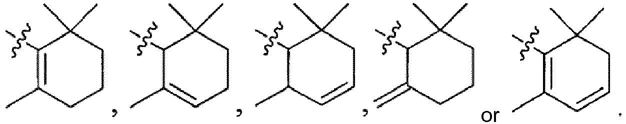

5. The odorant composition according to claim 4, **characterized in that** the compound of formula (I) is selected from at least one representative of the group formed of α-damascone, β-damascone, δ-damascone, γ-damascone, damascenone, cinnamaldehyde, hydroxycinnamaldehyde, iron, citral, ionones, α-isomethyl ionone, methyl cedryl ketone.

6. The odorant composition according to one of claims 1 to 5, wherein the odorant that contains thiol groups is an odorant having from 5 to 20 carbon atoms, preferably selected from (R)-thioterpineol, (S)-thioterpineol (p-menth-1-en-8-thiol), 2-(1-mercapto-1-methylethyl)-5-methyl-cyclohexanone, 4-methoxy-2-methyl-2-butanethiol, 3-mercapto-1-hexanol, 2-mercaptopropionic acid, 2-mercaptopropionic acid ethyl ester and 3-(methylthio)propyl mercaptoacetate.

7. An agent, **characterized in that** said agent contains at least one odorant composition according to one of claims 1 to 6, preferably in a total amount of from 0.001 to 99 wt.%, particularly preferably from 0.05 to 1 wt.%, based on the weight of the agent or adhesive, wherein the agent being a detergent or cleaning agent, air-care agent, cosmetic agent or adhesive.

8. The use of a washing or cleaning agent containing an odorant composition according to one of claims 1 to 6 for cleaning and/or conditioning textile fabrics, in particular in an automatic washing machine.

9. The use of an α,β-unsaturated compound selected from α,β-unsaturated ketones, α,β-unsaturated aldehydes or mixtures thereof for stabilizing an odorant that contains thiol groups.

10. The use according to claim 9, **characterized in that** the at least one α,β-unsaturated compound is an odorant.

11. The use according to claim 9 or 10, **characterized in that** the at least one α,β-unsaturated compound is a compound according to formula (I)
R¹-CH=CH-C(=O)R² (I)
where R¹ and R² each represent, independently of one another, hydrogen, a substituted or unsubstituted group containing alkyl, cycloalkyl, alkenyl, cycloalkenyl, aryl or acyl groups, or a polymer.

12. The use according to claim 11, **characterized in that**
(1) R¹ represents a substituted or unsubstituted alkyl group having from 1 to 10 carbon atoms, in particular a methyl group, and/or
(2) R² represents a group that contains an alkyl, cycloalkyl, alkenyl, cycloalkenyl, aryl or acyl group and has at least 5 carbon atoms, and in particular it comprises a cyclic, substituted alkenyl group having at least 5 carbon atoms, preferably

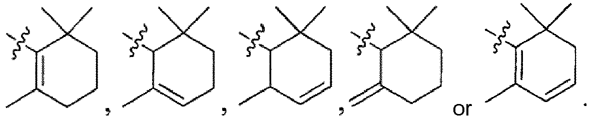

13. The use according to claim 12, **characterized in that** the compound of formula (I) is selected from at least one representative of the group formed of α-damascone, β-damascone, δ-damascone, γ-damascone, damascenone, cinnamaldehyde, hydroxycinnamaldehyde, iron, citral, ionones, α-isomethyl ionone, methyl cedryl ketone.

14. The use according to one of claims 9 to 13, wherein the odorant that contains thiol groups is an odorant having from 5 to 20 carbon atoms, preferably selected from (R)-thioterpineol, (S)-thioterpineol (p-menth-1-en-8-thiol), 2-(1-mercapto-1-methylethyl)-5-methyl-cyclohexanone, 4-methoxy-2-methyl-2-butanethiol, 3-mercapto-1-hexanol, 2-mercaptopropionic acid, 2-mercaptopropionic acid ethyl ester and 3-(methylthio)propyl mercaptoacetate.

## Revendications

1. Composition de substance odorante **caractérisée en ce que** la composition de substance odorante contient :
(i) au moins un composé α,β-insaturé choisi parmi les cétones α,β-insaturés, les aldéhydes α,β-insaturés ou des mélanges de ceux-ci ; et
(ii) au moins une substance odorante contenant des groupes thiols.
l'au moins un composé α,β-insaturé (i) et l'au moins une substance odorante contenant des groupes thiols étant employés dans un rapport molaire compris entre 5:1 et 1:1.

2. Composition de substance odorante selon la revendication 1, **caractérisée en ce que** l'au moins un composé α,β-insaturé est une substance odorante.

3. Composition de substance odorante selon la revendication 1 ou 2, **caractérisée en ce que** l'au moins un composé α,β-insaturé est un composé selon la formule (I)
R¹-CH=CH-C(=O)R² (I)
dans laquelle R¹ et R² représentent respectivement indépendamment l'un de l'autre de l'hydrogène, un résidu contenant des groupes alkyles, cycloalkyles, alcényles, cycloalcényles, aryles ou acyles non-substitués ou un polymère.

4. Composition de substance odorante selon la revendication 3, **caractérisé en ce que**
(1) R¹ représente un résidu alkyle substitué ou non-substitué comportant 1 à 10 atomes de carbone, en particulier un résidu méthyle, et/ou
(2) R² représente un résidu contenant un groupe alkyle, cycloalkyle, alcényle, cycloalcényle, aryle ou acyle comportant au moins 5 atomes de carbone, comportant en particulier un résidu alcényle cyclique, substitué, comportant 5 atomes de carbone, de préférence

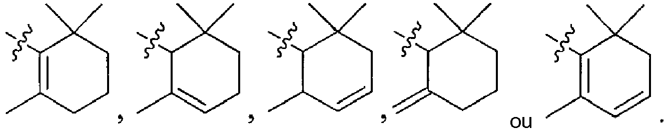

5. Composition de substance odorante selon la revendication 4, **caractérisé en ce que** le composé de la formule (I) est choisi parmi au moins un représentant du groupe composé de α-damascone, de β-damascone, de _{δ}-damascone, de γ-damascone, de damascénone, d'aldéhyde cinnamique, d'aldéhyde hydroxycinnamique, de fer, de citral, de janones, de α-isométhylionone, de méthylcédrylcétone.

6. Composition de substance odorante selon l'une des revendications 1 à 5, la substance odorante contenant des groupes thiol étant une substance odorante comportant 5-20 atomes de carbone, de manière préférée choisis parmi le (*R*)-, (*S*)-thioterpinol (p-menth-1-en-8-thiol), 2-(1-mercapto-1-méthyléthyl)-5-méthyl-cyclohexanone, 4-méthoxy-2-méthyl-2-butanethiol, 3-mercapto-1-hexanol, l'acide 2-mercaptopropionique et le 3-(méthylthio)propylmercaptoacétate.

7. Agent **caractérisé en ce que** l'agent contient au moins une composition de substance odorante selon l'une des revendications 1 à 6, de préférence dans une quantité totale de 0,001-99 % en poids, de manière particulièrement préférée de 0,05-1 % en poids, rapporté au poids de l'agent ou de la colle, l'agent étant un agent de lavage ou de nettoyage, un agent de parfum d'ambiance, un agent cosmétique ou une colle.

8. Utilisation d'un agent de lavage ou de nettoyage contenant une composition de substance odorante selon l'une des revendications 1 à 6 pour nettoyer et/ou conditionner d'étoffes textiles, en particulier dans une machine à laver automatique.

9. Utilisation d'un composé α,β-insaturé choisi parmi les cétones α,β-insaturés, les aldéhydes α,β-insaturés ou des mélanges de ceux-ci pour stabiliser une substance odorante contenant des groupes thiol.

10. Utilisation selon la revendication 9, **caractérisée en ce que** l'au moins un composé α,β-insaturé est une substance odorante.

11. Utilisation selon la revendication 9 ou 10, **caractérisée en ce que** l'au moins un composé α,β-insaturé est un composé selon la formule (I)
R¹-CH=CH-C(=O)R² (I)
dans laquelle R¹ et R² représentent respectivement indépendamment l'un de l'autre de l'hydrogène, un résidu contenant des groupes alkyles, cycloalkyles, alcényles, cycloalcényles, aryles ou acyles non-substitués ou un polymère.

12. Utilisation selon la revendication 11, **caractérisée en ce que**
(1) R¹ représente un résidu alkyle substitué ou non-substitué comportant 1 à 10 atomes de carbone, en particulier un résidu méthyle, et/ou
(2) R² représente un résidu contenant un groupe alkyle, cycloalkyle, alcényle, cycloalcényle, aryle ou acyle comportant au moins 5 atomes de carbone, comportant en particulier un résidu alcényle cyclique, substitué, comportant 5 atomes de carbone, de préférence

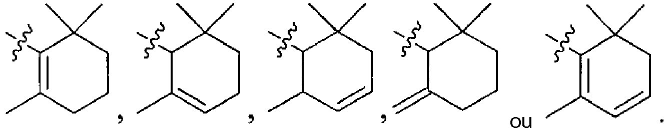

13. Utilisation selon la revendication 12, **caractérisée en ce que** le composé de la formule (I) est choisi parmi au moins un représentant du groupe composé de α-damascone, de β-damascone, de _{δ}-damascone, de γ-damascone, de damascénone, d'aldéhyde cinnamique, d'aldéhyde hydroxycinnamique, de fer, de citral, de janones, de α-isométhylionone, de méthylcédrylcétone.

14. Utilisation selon l'une des revendications 9 à 13, la substance odorante contenant des groupes thiol étant une substance odorante comportant 5-20 atomes de carbone, de manière préférée choisis parmi le (*R*)-, (*S*)-thioterpinol (p-menth-1-en-8-thiol), 2-(1-mercapto-1-méthyléthyl)-5-méthyl-cyclohexanone, 4-méthoxy-2-méthyl-2-butanethiol, 3-mercapto-1-hexanol, l'acide 2-mercaptopropionique et le 3-(méthylthio)propylmercaptoacétate.
